# EUROPEAN PATENT SPECIFICATION

(11) **EP 2 930 248 B1**
(45) Date of publication and mention of the grant of the patent: **19.04.2017**
(21) Application number: 15161333.8
(22) Date of filing: 27.03.2015
(51) Int. Cl.: C12Q 1/68

(54) **METHOD FOR PREDICTING SENSITIVITY OF OSTEOSARCOMA PATIENT TO ANTICANCER AGENT**
VERFAHREN ZUR VORHERSAGE DER EMPFINDLICHKEIT VON OSTEOSARKOMPATIENTEN GEGENÜBER EINEM ANTIKREBSMITTEL
PROCÉDÉ POUR PRÉDIRE LA SENSIBILITÉ D'UN PATIENT PRÉSENTANT UN OSTÉOSARCOME À UN AGENT ANTICANCÉREUX

(30) Priority: 31.03.2014 JP 2014074142; 19.01.2015 JP 2015007991
(43) Date of publication of application: 14.10.2015
(73) Proprietor: National Cancer Center, Tokyo 104-0045 (JP); Sysmex Corporation, Kobe-shi, Hyogo 651-0073 (JP)
(72) Inventor: Kubota, Daisuke, Tokyo 104-0045 (JP); Kawai, Akira, Tokyo 104-0045 (JP); Kondo, Tadashi, Tokyo 104-0045 (JP)
(74) Representative: Hoffmann Eitle

(56) References cited:
- WO-A1-2012/175711
- WO-A2-2011/135459
- YONG ZHOU ET AL: "miR-33a is up-regulated in chemoresistant osteosarcoma and promotes osteosarcoma cell resistance to cisplatin by down-regulating TWIST", JOURNAL OF EXPERIMENTAL & CLINICAL CANCER RESEARCH, BIOMED CENTRAL LTD, LONDON UK, vol. 33, no. 1, 27 January 2014 (2014-01-27), page 12, XP021179182, ISSN: 1756-9966, DOI: 10.1186/1756-9966-33-12
- JIANHUA HUANG ET AL: "MicroRNA-100 inhibits osteosarcoma cell proliferation by targeting Cyr61", TUMOR BIOLOGY, vol. 35, no. 2, 1 February 2014 (2014-02-01), pages 1095-1100, XP055207743, ISSN: 1010-4283, DOI: 10.1007/s13277-013-1146-8
- LI-HONG LIU ET AL: "miR-125b suppresses the proliferation and migration of osteosarcoma cells through down-regulation of STAT3", BIOCHEMICAL AND BIOPHYSICAL RESEARCH COMMUNICATIONS, vol. 416, no. 1-2, 1 December 2011 (2011-12-01), pages 31-38, XP055207938, ISSN: 0006-291X, DOI: 10.1016/j.bbrc.2011.10.117
- KEIICHIRO IIDA ET AL: "miR-125b develops chemoresistance in Ewing sarcoma/primitive neuroectodermal tumor", CANCER CELL INTERNATIONAL, BIOMED CENTRAL, LONDON, GB, vol. 13, no. 1, 4 March 2013 (2013-03-04), page 21, XP021141774, ISSN: 1475-2867, DOI: 10.1186/1475-2867-13-21
- ZHE ZHU ET AL: "MicroRNA-100 Resensitizes Resistant Chondrosarcoma Cells to Cisplatin through Direct Targeting of mTOR", ASIAN PACIFIC JOURNAL OF CANCER PREVENTION, vol. 15, no. 2, 30 January 2014 (2014-01-30), pages 917-923, XP055207922, ISSN: 1513-7368, DOI: 10.7314/APJCP.2014.15.2.917
- B SONG ET AL: "Mechanism of chemoresistance mediated by miR-140 in human osteosarcoma and colon cancer cells", ONCOGENE, vol. 28, no. 46, 7 September 2009 (2009-09-07), pages 4065-4074, XP055022150, ISSN: 0950-9232, DOI: 10.1038/onc.2009.274
- GUANGYI ZHAO ET AL: "MicroRNA-221 Induces Cell Survival and Cisplatin Resistance through PI3K/Akt Pathway in Human Osteosarcoma", PLOS ONE, vol. 8, no. 1, 23 January 2013 (2013-01-23), page e53906, XP055207691, DOI: 10.1371/journal.pone.0053906
- G. ZHOU ET AL: "MicroRNAs in osteosarcoma: From biological players to clinical contributors, a review", JOURNAL OF INTERNATIONAL MEDICAL RESEARCH, vol. 41, no. 1, 24 January 2013 (2013-01-24), pages 1-12, XP055207769, ISSN: 0300-0605, DOI: 10.1177/0300060513475959

## Description

### TECHNICAL FIELD

The present invention relates to a method for predicting sensitivity of a patient with osteosarcoma to an anticancer agent prior to administration of the anticancer agent.

### BACKGROUND ART

Osteosarcoma is a type of malignant primary bone tumour and is a disease in which tumour cells produce bone and osteoid matrix. A part of the femur and the tibia proximal to the knee joint is the most common site where osteosarcoma occurs and a part of the humerus proximal to the shoulder joint is the second. A definitive diagnosis of osteosarcoma is made by a pathological examination of a biopsy sample collected from the site where osteosarcoma may have occurred. When the diagnosis of osteosarcoma is made, preoperative chemotherapy with an anticancer agent is generally given over about 6 months in order to minimise the tumour and thus the resection area before an operation for resection. It is suggested that about 80% of patients who responded to anticancer agents may exhibit complete remission while about 80% of patients who did not respond to anticancer agents may die within 5 years. If patients do not respond to anticancer agents during preoperative chemotherapy, immediate resection of the tumour by surgery is effective.

When a patient with osteosarcoma responds to an anticancer agent, the patient may undergo resection after preoperative chemotherapy. However, chemotherapy given to osteosarcoma which does not respond to an anticancer agent is useless in the end and the disease may even progress during the useless chemotherapy. If sensitivity of osteosarcoma to an anticancer agent can be predicted prior to preoperative chemotherapy, it is possible to avoid an overdose during preoperative chemotherapy and make a decision for early resection of the tumour. Thus, in order to preliminarily make a decision for an appropriate regimen, development of the method is required that allows the prediction of sensitivity of osteosarcoma to anticancer agents.

The inventors of the present invention found that a protein molecule called peroxiredoxin 2 can be utilized as a biomarker for the prediction of sensitivity of osteosarcoma to anticancer agents (see Japanese Unexamined Patent Application Publication No. 2011-63524). Recently, it is reported that microRNAs (hereinafter also referred to as "miRNAs") are involved in oncogenesis, prognosis of cancer and resistance to anticancer agents. For example, it is reported that the drug resistance is correlated with the shift in expression levels of miRNA-100 and miR-125b in various cancer cells (see Feng B et al., MiR-100 resensitizes docetaxel-resistant human lung adenocarcinoma cells (SPC-A1) to docetaxel by targeting Plk1, Cancer Lett., 2012, 317: 184-91; Dai Y et al., MicroRNA expression profiles of head and neck squamous cell carcinoma with docetaxel-induced multidrug resistance, Head Neck., 2011, 33: 786-91; Schotte D et al., MicroRNA characterize genetic diversity and drug resistance in pediatric acute lymphoblastic leukemia, Haematologica. 2011, 96: 703-11; Hatano Yet al., miR-125b develops chemoresistance in Ewing sarcoma/primitive neuroectodermal tumor, Cancer Cell Int. 2013, 13: 21; Cui EH et al., Serum microRNA 125b as a diagnostic or prognostic biomarker for advanced NSCLC patients receiving cisplatin-based chemotherapy, Acta Pharmacol Sin. 2013, 34: 309-13; Wang H et al., Circulating MiR-125b as a marker predicting chemoresistance in breast cancer, PLoS One. 2012, 7: e34210; Zhang H et al., Upregulation of microRNA-125b contributes to leukemogenesis and increases drug resistance in pediatric acute promyelocytic leukemia, Mol Cancer. 2011, 10: 108; Shi L et al., MicroRNA-125b-2 confers human glioblastoma stem cells resistance to temozolomide through the mitochondrial pathway of apoptosis, Int J Oncol. 2012, 40: 119-29; Kong F et al., miR-125b confers resistance of ovarian cancer cells to cisplatin by targeting pro-apoptotic Bcl-2 antagonist killer 1, Technolog Med Sci. 2011, 31: 543-9; Zhou M et al., MicroRNA-125b confers the resistance of breast cancer cells to paclitaxel through suppression of pro-apoptotic Bcl-2 antagonist killer 1 (Bak1) expression, J Biol Chem. 2010, 285: 21496-507).

However, none of the above references has reported correlation between the expression levels of miRNA-100 and miRNA-125b in osteosarcoma and sensitivity to anticancer agents administered after the expression levels are examined. Further, in these references, the expression levels of miRNA-100 and miRNA-125b are determined in cell lines which have acquired resistance to anticancer agents after the anticancer agents being given (cell lines with acquired resistance). Namely, these references merely disclose the profile of the expression levels of miRNA-100 and miRNA-125b in cells which have acquired resistant to anticancer agents, and thus the correlation between the expression levels of miRNA-100 and miRNA-125b in cells which have not received anticancer agents and sensitivity of the cells to the anticancer agent is not disclosed.

### SUMMARY OF THE INVENTION

As described above, the inventors of the present invention found biomarkers which may allow the prediction of sensitivity to anticancer agents. However, other markers than those described above have not been found and in order to allow a more accurate prediction, there is a need for developing novel markers. Therefore an object of the present invention is to provide a method for predicting sensitivity of an osteosarcoma patient to an anticancer agent before administration of the anticancer agent.

The inventors of the present invention have found that, among osteosarcoma cells collected from osteosarcoma patients to whom an anticancer agent has not been administered, the expression levels of miRNA-100 and miRNA-125b are higher in the cells which exhibit resistance to the anticancer agent which is administered afterwards than in the cells which exhibit sensitivity to the anticancer agent, thereby completing the present invention.

Thus the present invention provides a method for predicting sensitivity of an osteosarcoma patient to an anticancer agent, comprising the steps of:
extracting RNA from a biological sample containing osteosarcoma cells collected from the osteosarcoma patient to whom the anticancer agent is not administered;
obtaining a value indicating an expression level of at least one of miRNA-100 and miRNA-125b in the extracted RNA; and
based on the obtained value indicating the expression level, predicting sensitivity of the osteosarcoma patient to the anticancer agent.

According to the present invention, sensitivity of an osteosarcoma patient to the anticancer agent can be predicted before administration of the anticancer agent. Therefore the present invention can assist to establish appropriate therapeutic regimens for osteosarcoma patients before preoperative chemotherapy.

### BRIEF DESCRIPTION OF THE DRAWINGS

Fig. 1A is a graph showing distribution of expression levels of miRNA-100 in a group of patients who responded to preoperative chemotherapy and in a group of patients who did not respond to preoperative chemotherapy;
Fig. 1B is a graph showing distribution of expression levels of miRNA-125b in a group of patients who responded to preoperative chemotherapy and in a group of patients who did not respond to preoperative chemotherapy;
Fig. 2A is a graph showing distribution of expression levels of miRNA-100 in a group of patients who responded to preoperative chemotherapy and in a group of patients who did not respond to preoperative chemotherapy;
Fig. 2B is a graph showing distribution of expression levels of miRNA-125b in a group of patients who responded to preoperative chemotherapy and in a group of patients who did not respond to preoperative chemotherapy;
Fig. 3A is an ROC curve obtained when sensitivity of osteosarcoma patients to preoperative chemotherapy was predicted based on the expression level of miRNA-100;
Fig. 3B is an ROC curve obtained when sensitivity of osteosarcoma patients to preoperative chemotherapy was predicted based on the expression level of miRNA-125b;
Fig. 4 is a schematic view of an example of a determination device for providing information on the prediction of sensitivity of an osteosarcoma patient to an anticancer agent;
Fig. 5 is a block diagram showing the functionality configuration of the determination device of Fig. 4;
Fig. 6 is a block diagram showing the hardware configuration of the determination device shown in Fig. 4;
Fig. 7 is a flow chart of determination for providing information on the prediction of sensitivity of an osteosarcoma patient to an anticancer agent using the determination device shown in Fig. 4; and
Fig. 8 is a photographic image showing the expression level of a proapoptotic factor, BAK1, analyzed by western blotting.

### MODES FOR CARRYING OUT THE INVENTION

In the method for predicting sensitivity of an osteosarcoma patient to an anticancer agent according to the present embodiment (hereinafter also merely referred to as "method"), osteosarcoma cells are not particularly limited as to morphology, growth rate, malignancy and the like of the cells. Osteosarcoma may be classified into conventional central osteosarcoma and subtypes of osteosarcoma. It is known that subtypes of osteosarcoma include intraosseous well-differentiated (low-grade) osteosarcoma, round-cell osteosarcoma, perosteal (juxtacortical) osteosarcoma, periosteal osteosarcoma and high-grade surface osteosarcoma. The method according to the present embodiment can be applied to cells derived from any type of osteosarcoma as far as they have not received an anticancer agent; however the method is particularly suitable for cells derived from conventional central osteosarcoma.

The method according to the present embodiment comprises the step of extracting RNA from a biological sample containing osteosarcoma cells collected from an osteosarcoma patient to whom the anticancer agent is not administered. As shown in Reference Example 2 described hereinbelow, administration of an anticancer agent may affect the expression level of miRNA-125b in osteosarcoma cells, and therefore the biological sample is desirably derived from a patient to whom the anticancer agent is not administered. The biological sample is not particularly limited as far as it contains osteosarcoma cells collected from an osteosarcoma patient to whom the anticancer agent is not administered and is preferably a clinical specimen collected from an osteosarcoma patient to whom the anticancer agent is not administered. The osteosarcoma patient is preferably a patient who has not received anticancer agent therapy after the diagnosis of osteosarcoma. As far as the osteosarcoma patient has not received any anticancer agent, age and sex of the patient, type of osteosarcoma, diseased site, disease stage and the like are not particularly limited.

Examples of a clinical specimen collected from an osteosarcoma patient to whom the anticancer agent is not administered include cells and tissues collected from diseased sites of osteosarcoma by surgery or biopsy, blood, serum, plasma, lymph fluid and the like. Among these, cells, tissues and blood are preferred. The biological sample may be cells or tissues obtained by culturing osteosarcoma cells or tissues obtained from the patient. Further, the biological sample may be a formalin-fixed paraffin-embedded (FFPE) sample of osteosarcoma tissue from the patient.

RNA can be extracted from a biological sample according to well known methods in the art. For example, total RNA can be extracted by mixing a biological sample with a lysis buffer containing guanidium thiocyanate and a surfactant and subjecting the obtained mixture to physical treatment (stirring, homogenization, ultrasonication and the like) to release RNA from the biological sample into the mixture. Preferably, the mixture is made acidic by addition of sodium acetate and the like, phenol and chloroform are further added to the mixture which is then centrifuged and the resulting aqueous layer containing RNA is collected. It is more preferable that RNA to be subjected to the subsequent measurement step is collected and purified from the aqueous layer by alcohol precipitation and the like methods. The lysis buffer may be a commercial RNA extraction reagent such as ISOGEN II (Nippon Gene, Co., Ltd.). RNA may be extracted and purified from a biological sample with a commercial reagent kit for extraction of total RNA containing miRNA such as the miRNeasy kit (QIAGEN) and the High Pure miRNA Isolation Kit (Roche).

The method according to the present embodiment comprises the step of obtaining a value indicating an expression level of at least one of miRNA-100 and miRNA-125b in RNA extracted from the biological sample. In the present embodiment, the value indicating the expression level may be the measured expression level *per se* of the miRNA or a value derived from the measured level.

In the art, miRNA-100 and miRNA-125b may also be referred to as miR-100 and miR-125b, respectively. The base sequences *per se* of miRNA-100 and miRNA-125b are well known and can be obtained from well known databases such as the one provided by the National Center for Biotechnology Information (NCBI) (http://www.ncbi.nlm.nih.gov/) and miRBase (http://microrna.sanger.ac.uk/). The base sequences of miRNA-100 and miRNA-125b are herein represented by SEQ ID NOs: 1 and 2, respectively.

In the present embodiment, the value indicating the expression level of miRNA-100 and miRNA-125b may be the amount of the miRNAs *per se* or the amount of a substance reflecting the amount of the miRNAs. Examples of the substance reflecting the amount of the miRNAs include cDNA obtained by reverse transcription of miRNA and cRNA obtained by IVT amplification of the cDNA. As the amount of miRNA in total RNA is extremely low, it is preferable that the value indicating the expression level of miRNA is the amount of cDNA or cRNA derived from the miRNA. cDNA derived from miRNA may be synthesized with specific primers complementary to the sequence of the miRNA or obtained by subjecting total RNA containing the miRNA to 3'-polyadenylation and synthesizing cDNA with an oligo(dT) primer. cDNA may alternatively be synthesized from miRNA with a commercial reagent kit such as the TaqMan^{®} MicroRNA RT Kit (Applied Biosystems) or the Mir-X^{®} miRNA First-Strand Synthesis Kit (Takara Bio Inc.).

In the present embodiment, the expression level of the miRNA may be measured according to any well known methods in the art without limitation. Examples of the measurement methods include a method utilizing hybridization with a probe, a method utilizing nucleic acid amplification and a method utilizing mass spectrometry. Examples of the method utilizing hybridization with a probe include microarray technique, northern hybridization, RNase protection assay and the like. Examples of the method utilizing nucleic acid amplification include quantitative reverse transcription PCR (qRT-PCR), quantitative reverse transcription LAMP (qRT-LAMP), next-generation sequencing (high-throughput sequencing) and the like. qRT-PCR using a TaqMan^{®} probe may also be mentioned. Among these, qRT-PCR is particularly preferred.

The expression level of the miRNA may alternatively be measured with a commercial reagent kit for miRNA quantification comprising primers for reverse transcription of miRNA-100 and miRNA-125b and specific primer sets for amplification of the resulting cDNAs such as TaqMan^{®} MicroRNA Assays.

A microarray for measurement of the expression level of the miRNA is not particularly limited as far as it is a chip including an appropriate substrate onto which a nucleic acid probe specifically hybridizing the miRNA or cDNA or cRNA derived therefrom (hereinafter the molecules are collectively referred to as "target nucleic acid molecule"). The probe may be appropriately designed on the basis of the base sequence of the miRNA and preferably includes the full-length sequence of the miRNA. Such a microarray can be prepared according to methods well known in the art. A commercial microarray may also be used as far as it comprises a probe specifically hybridizing the target nucleic acid molecule.

The expression "specifically hybridizing" as used herein means an ability of a probe to hybridize the target nucleic acid molecule under the stringent condition. The term "stringent condition" refers to a condition under which a probe can hybridize the target nucleic acid molecule at an extent that is detectably higher than hybridization of the probe to a nucleic acid molecule other than the target nucleic acid molecule (e.g. at least 2 times of the background). The stringent condition is generally sequence-dependent and varies according to various environments. Generally, the stringent condition is selected so as to be at about 5°C lower than the thermal melting point (Tm) of a specific sequence at a defined ionic strength and pH. Tm is a temperature at which 50% of probes complementary to the base sequence of a target nucleic acid molecule are equilibrated and hybridize to the target nucleic acid molecule under a defined ionic strength, pH and nucleic acid composition. The stringent condition may be the one well known in the art used for hybridization of polynucleotides. Specific examples of the stringent condition include at least about 30°C, pH of 7.0 to 9.0 and the salt concentration of lower than about 1.5 M Na-ion concentration, more specifically about 0.01 to 1.0 M Na-ion (or other salt) concentration. For example, the stringent condition for microarray may include hybridization at 37°C in 50% formamide, 1 M NaCl and 1% SDS and washing at 60 to 65°C in 0.1 x SSC.

When the expression level is measured using a microarray, the target nucleic acid molecule is preferably labelled with a labelling substance well known in the art. Labelling of the target nucleic acid molecule facilitates measurement of a signal from a probe on a microarray. In the present embodiment, miRNA extracted from a biological sample may be labelled or cDNA or cRNA derived from the miRNA may be labelled. Examples of the labelling substance include fluorescence substances, haptens such as biotin, radioactive substances and the like. Examples of the fluorescence substances include Cy3, Cy5, FITC, Alexa Fluor™ and the like. Methods for labelling miRNA, cDNA and cRNA with the labelling substance are well known in the art.

In measurements using a microarray, the obtained value indicating the expression level of the miRNA is a signal intensity (e.g. fluorescence intensity, luminescence intensity, variation in the amount of current) originating from hybridization between the target nucleic acid molecule and a probe on the microarray. The value indicating the expression level of the miRNA may alternatively be the quantitative value of the miRNA obtained on the basis of the signal intensity and a calibration curve. The signal can be detected on a scanner comprised in a conventional microarray analyzer.

Any primer can be used for nucleic acid amplification as far as it can specifically hybridize to the target nucleic acid molecule and amplify the molecule. The stringent condition for PCR may be, for example, pH 7.0 to 9.0, 0.01 to 0.1 M Tris HCl, 0.05 to 0.15 M potassium (or other salt) ion concentration and at least about 55°C. The primer may be appropriately designed on the basis of the base sequence of the miRNA. The primer is preferably designed according to the type of nucleic acid amplification technique. The primer can be produced according to well known nucleic acid synthesis methods.

The primer may be labelled with a labelling substance well known in the art. The primer may be labelled with a radioactive element or a non-radioactive molecule. Examples of the radioactive isotope include ³²P, ³³P, ³⁵S, 3H and ¹²⁵I. Examples of the non-radioactive substance include ligands such as biotin, avidin, streptavidin and digoxigenin, haptens, dyes and luminescence reagents such as chemiluminescence, bioluminescence, fluorescence and phosphorescence reagents.

In measurements based on nucleic acid amplification, examples of the value indicating the expression level of the miRNA include an optical value (fluorescence intensity, turbidity, absorbance, etc.) obtained by measuring a reaction solution after amplification, the number (or duration) of cycles when the measured optical value reaches to a certain criteria, the miRNA quantity obtained from the measured optical value (or the number of cycles) and a calibration curve. The certain criteria can be appropriately established according to the type of the value indicating the expression level. When the value indicating the expression level is the number of cycles, for example, the certain criteria may be the amount of change of the fluorescence intensity during logarithmic nucleic acid amplification. The measured values for miRNA-100 and miRNA-125b may be normalized with the measured expression level of a small RNA which serves as an internal control.

The method according to the present embodiment comprises the step of predicting sensitivity of osteosarcoma cells contained in a biological sample collected from an osteosarcoma patient to whom an anticancer agent is not administered to the anticancer agent based on the value indicating the expression level obtained as above.

In the present embodiment, it is predicted whether sensitivity to the anticancer agent of the osteosarcoma patient who has not received the anticancer agent is high or low when the anticancer agent is administered to the patient in a later stage. For example, when it is predicted that sensitivity to an anticancer agent is high, it can mean that the viability of osteosarcoma cells after administration of the anticancer agent to the cells is predicted to be less than 10% as evaluated according to the Huvos grading system. To the contrary, when it is predicted that sensitivity to an anticancer agent is low, it can mean that the viability of osteosarcoma cells after administration of the anticancer agent to the cells is predicted to be at or higher than 10% as evaluated according to the Huvos grading system.

In the present embodiment, the prediction of sensitivity of an osteosarcoma patient to whom an anticancer agent is not administered to the anticancer agent can be construed as the prediction of sensitivity of osteosarcoma cells collected from the osteosarcoma patient to the anticancer agent. Namely, when it is predicted that sensitivity to an anticancer agent is high, it means that it is predicted that osteosarcoma cells collected from the osteosarcoma patient are sensitive to the anticancer agent. To the contrary, when it is predicted that sensitivity to an anticancer agent is low, it means that it is predicted that osteosarcoma cells collected from the osteosarcoma patient are resistant to the anticancer agent.

In the present embodiment, it is preferable that in the step of predicting, the obtained value indicating the expression level is compared with a predetermined threshold and sensitivity of the osteosarcoma patient to the anticancer agent is predicted based on the comparison result. Specifically, when the comparison result shows that the value indicating the expression level is at or higher than a predetermined threshold, it is predicted that sensitivity of the osteosarcoma patient to the anticancer agent is low. To the contrary, when the comparison result shows that the value indicating the expression level is less than a predetermined threshold, it is predicted that sensitivity of the osteosarcoma cells to the anticancer agent is high.

The predetermined threshold is not particularly limited and may be empirically established by accumulating data on, for example, sensitivity of osteosarcoma patients to whom an anticancer agent is not administered to the anticancer agent. The threshold may alternatively be established as follows. First, biological samples are collected from more than one osteosarcoma patient who has not received an anticancer agent. After administration of treatment with the anticancer agent to the osteosarcoma patients from who the biological samples have been collected, tumour tissues are resected from the osteosarcoma patient. The resected tumour tissues are pathologically examined and sensitivity of the osteosarcoma patients to the anticancer agent is examined. The biological samples are then divided into samples of osteosarcoma patients who responded to the anticancer agent and samples of osteosarcoma patients who did not respond to the anticancer agent and the expression levels of miRNA-100 and miRNA-125b are obtained from the respective samples. Based on the obtained levels, a threshold is established so that the samples of osteosarcoma patients who responded to the anticancer agent can be differentiated from the samples of osteosarcoma patients who did not respond to the anticancer agent.

In the present embodiment, it is preferable that in the step of predicting, sensitivity to an anticancer agent for use in treatment of osteosarcoma is predicted. The anticancer agent is selected from anti-metabolites, anticancer antibiotics, anticancer plant alkaloids, platinum agents and alkylating agents. Examples of anti-metabolites include methotrexate, tegafur, carmofur, 5-fluorouracil, cytarabine, gemcitabine, capecitabine, fludarabine, pemetrexed, pentostatin, enocitabine and the like. Examples of anticancer antibiotics include doxorubicin, daunorubicin, bleomycin, aclarubicin, idarubicin, epirubicin, pirarubicin, peplomycin, mitoxantrone, actinomycin D, mitomycin C and the like. Examples of anticancer plant alkaloids include docetaxel, paclitaxel, irinotecan, nogitecan, etoposide, sobuzoxane, vinorelbine, vindesine, vincristine, vinblastine and the like. Examples of platinum agents include cisplatin, carboplatin, oxaliplatin, nedaplatin and the like. Examples of alkylating agents include ifosfamide, cyclophosphamide, melphalan, busulfan, nimustine, temozolomide, dacarbazine and the like.

Among the above anticancer agents, the method according to the present embodiment is particularly suitable for the prediction of sensitivity to at least one anticancer agent selected from methotrexate, doxorubicin, cisplatin and ifosfamide. Particularly, the method according to the present embodiment is suitable for the prediction of sensitivity to at least one anticancer agent selected from methotrexate, doxorubicin and cisplatin. These three anticancer agents, methotrexate, doxorubicin and cisplatin, are generally combined in preoperative chemotherapy of osteosarcoma.

An embodiment of the present invention also encompasses a system suitable for provision of information on the prediction of sensitivity of an osteosarcoma patient to an anticancer agent. For example, the system may be as follows.

A system suitable for provision of information on the prediction of sensitivity of an osteosarcoma patient to an anticancer agent comprising a computer containing a processor and a memory controlled by the processor, wherein the memory comprises a computer program for enabling the computer to carry out the steps of:
obtaining a value indicating an expression level of at least one of miRNA-100 and miRNA-125b in RNA derived from a biological sample containing osteosarcoma cells collected from the osteosarcoma patient to whom the anticancer agent is not administered; and
providing information on the prediction of sensitivity of the osteosarcoma patient to the anticancer agent based on the obtained value indicating the expression level.

An embodiment of the present invention also encompasses a computer program product for enabling a computer to carry out provision of information on the prediction of sensitivity of an osteosarcoma patient to whom an anticancer agent is not administered to the anticancer agent. For example, the computer program product may be as follows.

A computer program product for enabling a computer to carry out provision of information on the prediction of sensitivity of an osteosarcoma patient to an anticancer agent comprising a computer readable medium, wherein the medium comprises a computer program for enabling the computer to carry out the steps of:
obtaining a value indicating an expression level of at least one of miRNA-100 and miRNA-125b in RNA derived from a biological sample containing osteosarcoma cells collected from the osteosarcoma patient to whom the anticancer agent is not administered; and
providing information on the prediction of sensitivity of the osteosarcoma patient to the anticancer agent based on the obtained value indicating the expression level.

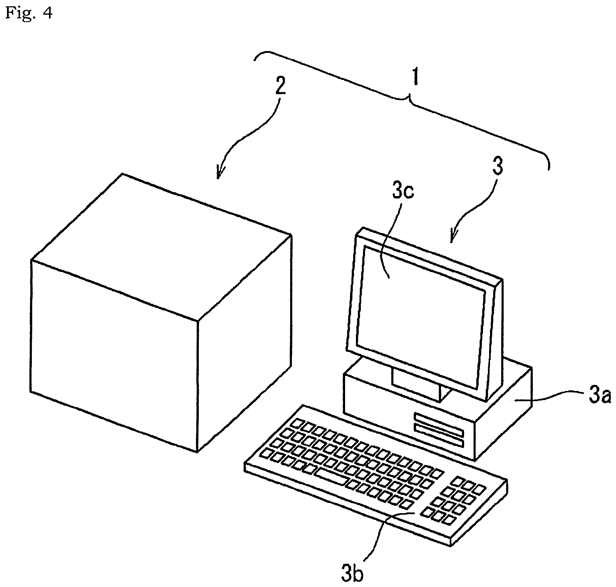

An embodiment of a suitable device for carrying out the method according to the present embodiment is illustrated hereinafter by referring to figures. However, the present invention is not limited only to this embodiment. Fig. 4 is a schematic view of an example of a determination device for providing information on sensitivity of an osteosarcoma patient to an anticancer agent. The determination device 1 shown in Fig. 4 comprises a measurement device 2 and a computer system 3 connected to the measurement device 2.

In the present embodiment, the measurement device 2 is a real-time PCR instrument. The measurement device 2 obtains the value indicating the expression level of miRNA-100 and/or miRNA-125b. The measurement device 2 onto which a measurement sample containing RNA prepared from a biological sample and reagents necessary for qRT-PCR is mounted obtains a measured optical value derived from an amplification product from miRNA-100 and/or miRNA-125b in the measurement sample and sends the obtained value to the computer system 3.

When the value indicating the expression level of miRNA-100 and/or miRNA-125b is analyzed by microarray technique, the measurement device 2 is a microarray scanner. In this case, the measurement device 2 onto which a microarray after being contacted to a target nucleic acid molecule derived from a biological sample is mounted detects a signal based on specific hybridization between a probe on the microarray and the target nucleic acid molecule. The measurement device 2 then obtains signal intensity data from the probe and sends the obtained data to the computer system 3.

The computer system 3 comprises a computer main body 3a, an input device 3b and a display 3c which displays specimen information, determination results and the like. The computer system 3 receives the value indicating the expression level from the measurement device 2. The processor in the computer system 3 executes, based on the value indicating the expression level, a program for providing information on the prediction of sensitivity of an osteosarcoma patient to an anticancer agent.

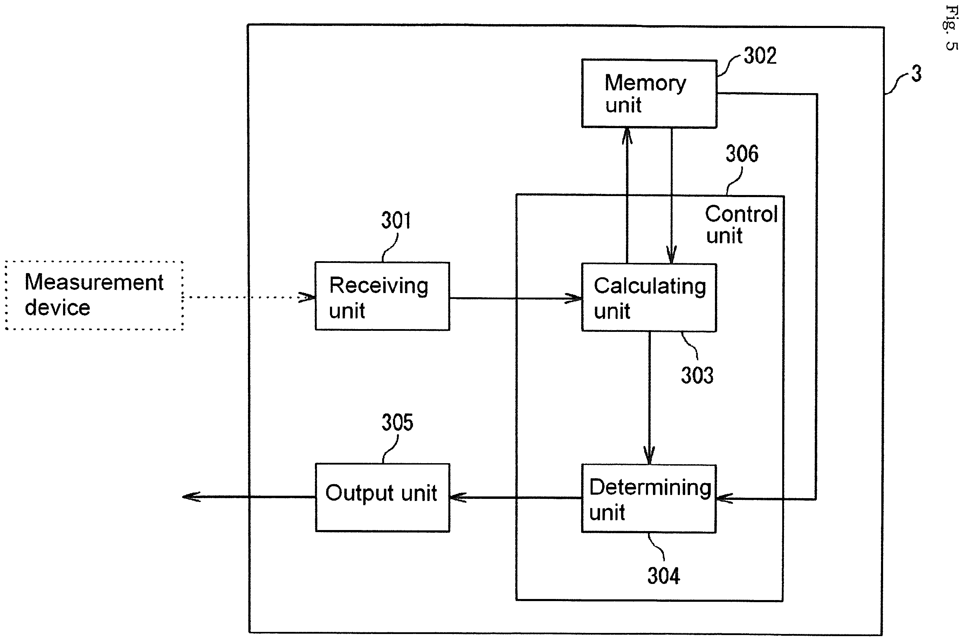

Fig. 5 is a block diagram showing the functionality configuration of the determination device shown in Fig. 4. As shown in Fig. 5, the computer system 3 comprises a receiving unit 301, a memory unit 302, a calculating unit 303, a determining unit 304 and an output unit 305. The receiving unit 301 is communicably connected to the measurement device 2 though a network. The calculating unit 303 and the determining unit 304 are included in a control unit 306.

The receiving unit 301 obtains information from the measurement device 2. The memory unit 302 stores a predetermined threshold and a formula for calculating the value indicating the expression level from the measured optical value. The calculating unit 303 calculates the value indicating the expression level of miRNA-100 and/or miRNA-125b from information obtained at the receiving unit 301 according to the formula stored in the memory unit 302. The determining unit 304 determines whether or not the value indicating the expression level calculated at the calculating unit 303 is lower than the predetermined threshold stored at the memory unit 302. The output unit 305 outputs the determination result from the determining unit 304 as information on the prediction of sensitivity of an osteosarcoma patient to an anticancer agent (e.g. whether or not sensitivity of the osteosarcoma patient to the anticancer agent is high or low).

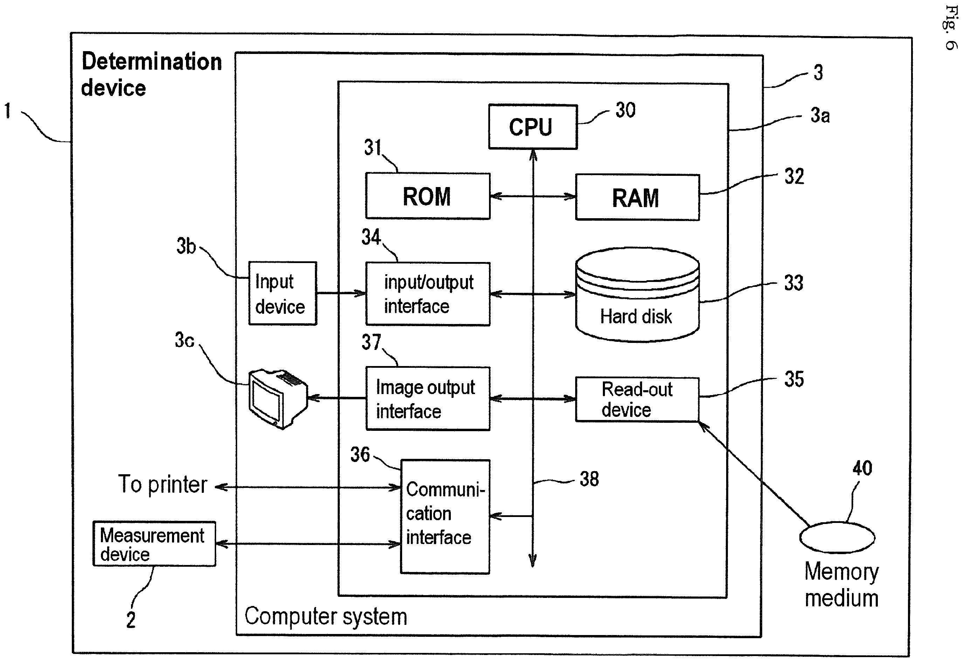

Fig. 6 is a block diagram showing the hardware configuration of the determination device shown in Fig. 4. As shown in Fig. 6, the computer main body 3a comprises a CPU (Central Processing Unit) 30, ROM (Read Only Memory) 31, RAM (Random Access Memory) 32, a hard disk 33, an input/output interface 34, a read-out device 35, a communication interface 36 and an image output interface 37. The CPU 30, ROM 31, RAM 32, the hard disk 33, the input/output interface 34, the read-out device 35, the communication interface 36 and the image output interface 37 are connected via a bus 38 so as to be able to communicate with each other.

The CPU 30 can execute a computer program stored in ROM 31 and a computer program loaded with RAM 32. When the CPU 30 executes the application program, the functional blocks described above may be executed. Accordingly the computer system serves as a terminal that is a determination device for providing information on the prediction of sensitivity of an osteosarcoma patient to an anticancer agent.

ROM 31 is made up with mask ROM, PROM, EPROM, EEPROM or the like. ROM 31 stores the computer program executed by the CPU 30 and data used for the execution.

RAM 32 is made up with SRAM, DRAM or the like. RAM 32 is used for readout of the computer programs stored in ROM 31 and the hard disk 33. RAM 32 is also utilized as a work area when the CPU 30 executes these computer programs.

An operating system to be executed by the CPU 30, computer programs such as application programs (the computer program for providing information on the prediction of sensitivity of an osteosarcoma patient to an anticancer agent) and data for executing the computer programs are installed on the hard disk 33.

The read-out device 35 is made up with a flexible disk drive, a CD-ROM drive, a DVD-ROM drive or the like and can read out the computer program or data stored on a portable memory medium 40.

The input/output interface 34 is made up with a serial interface such as USB, IEEE 1394 and RS-232C, a parallel interface such as SCSI, IDE and IEEE 1284 and an analogue interface formed by a D/A converter, an A/D converter or the like. The input/output interface 34 is connected to the input device 3b such as a keyboard and a mouse. A user can input data into the computer main body 3a by means of the input device 3b.

The communication interface 36 is, for example, an Ethernet^{®} interface. The computer system 3 can send printing data to a printer via the communication interface 36. The communication interface 36 is connected to the measurement device 2. The CPU 30 sends/receives signals and data to/from the measurement device 2 through the communication interface 36.

The image output interface 37 is connected to the display 3c made up with a LCD, a CRT and the like. Accordingly the display 3c can output an image signal according to image data provided by the CPU 30. The display 3c displays an image (on a screen) according to the input image signal.

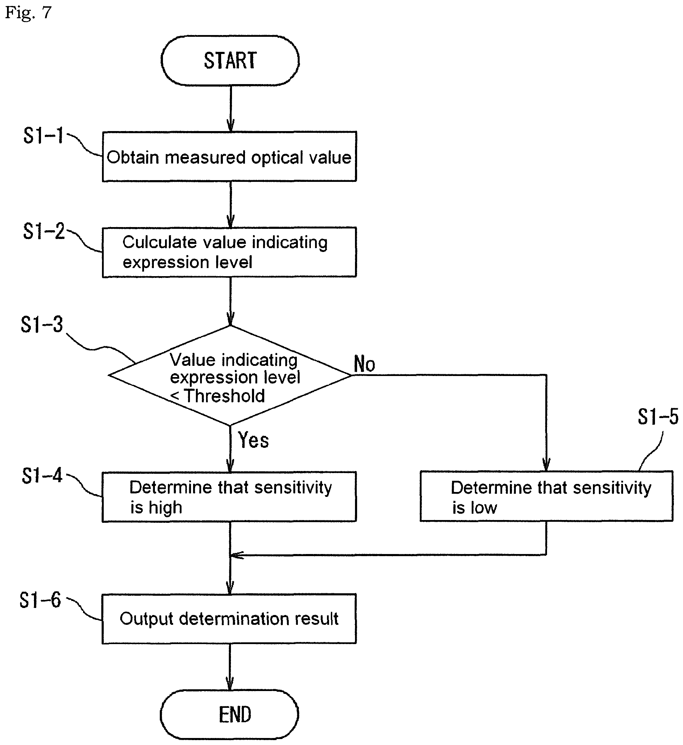

The process operations carried out by the determination device 1 for providing information on the prediction of sensitivity of an osteosarcoma patient to an anticancer agent are illustrated hereinafter. Fig. 7 is a flow chart for providing information on the prediction of sensitivity of an osteosarcoma patient to an anticancer agent using the determination device shown in Fig. 4. An example is herein illustrated in which the value indicating the expression level of miRNA-100 and/or miRNA-125b is obtained from a measured optical value sent from the measurement device 2 and the prediction is carried out with the obtained value indicating the expression level. However, the present invention is not limited only to this embodiment.

In the step S1-1, the receiving unit 301 in the determination device 1 receives from the measurement device 2 a measured optical value. In the next step S1-2, the calculating unit 303 calculates from the measured optical value received at the receiving unit 301 the value indicating the expression level of miRNA-100 and/or miRNA-125b according to the formula for calculating the value indicating the expression level stored in the memory unit 302.

In the subsequent step S1-3, the determining unit 304 determines whether or not the value indicating the expression level calculated at the calculating unit 303 is lower than the threshold stored in the memory unit 302. When the value indicating the expression level is lower than the predetermined threshold, the determining unit 304 in the step S1-4 sends a determination result indicating that sensitivity of the osteosarcoma patient to the anticancer agent is predicted to be high to the output unit 305. When on the other hand the value indicating the expression level is not lower than the predetermined threshold (i.e., the value indicating the expression level is at or higher than the predetermined threshold), the determining unit 304 in the step S1-5 sends a determination result indicating that sensitivity of the osteosarcoma patient to the anticancer agent is predicted to be low to the output unit 305.

In the step S1-6, the output unit 305 outputs the determination result as information on the prediction of sensitivity of the osteosarcoma patient to the anticancer agent, so that the display 3c displays the result and/or the printer prints out the result. Accordingly, the determination device can provide, to a physician or the like, information assisting the physician or the like to determine therapeutic regimen of the osteosarcoma patient before initiation of therapy using the anticancer agent.

The present invention is specifically described hereinafter by way of Examples which do not limit the present invention.

### Examples

### Example 1: Identification of novel biomarkers

### (1) Preparation of biological samples

Eight osteosarcoma patients after receiving preoperative chemotherapy were divided into, according to the Huvos Grading System, a group of patients who responded to the preoperative chemotherapy (4 patients with Case Nos. 1 to 4) and a group of patients who did not respond to the preoperative chemotherapy (4 patients with Case Nos. 5 to 8) (see Table 1). Anticancer agents (neo-adjuvants) used in the preoperative chemotherapy were methotrexate, doxorubicin and cisplatin. In Example 1, the biological samples used were frozen tissue samples previously obtained from the 8 osteosarcoma patients by incisional biopsy before the start of the preoperative chemotherapy. The group of patients who responded to the preoperative chemotherapy corresponded to Huvos grade 1 or 2 and had the viability of osteosarcoma cells after the preoperative chemotherapy of less than 10% as measured by pathological examination of specimens obtained during operations. The group of patients who did not respond to the preoperative chemotherapy corresponded to the Huvos grade 3 or 4 and had the viability of osteosarcoma cells after the preoperative chemotherapy of at or higher than 10% as measured by pathological examination of specimens obtained during operations.

**Table 1**

| Case No. | Gender | Age | Location | Histologic type | neo-adjuvant | Viable cells (%)^{A} |
|---|---|---|---|---|---|---|
| Case1 | female | 11 | tibia | osteoblastic | MTX + DOX + CDDP | 0% |
| Case2 | male | 18 | tibia | osteoblastic | MTX + DOX + CDDP | 9% |
| Case3 | male | 15 | tibia | osteoblastic | MTX + DOX + CDDP | 0% |
| Case4 | female | 13 | femur | osteoblastic | MTX + DOX + CDDP | 0% |
| Case5 | female | 10 | femur | osteoblastic | MTX + DOX + CDDP | 70% |
| Case6 | male | 25 | tibia | osteoblastic | MTX + DOX + CDDP | 40% |
| Case7 | male | 12 | femur | osteoblastic | MTX + DOX + CDDP | 20% |
| Case8 | female | 16 | femur | osteoblastic | MTX + DOX + CDDP | 22% |

| | | | | | | |
|---|---|---|---|---|---|---|
| A: Probability of viable cells were pathologically evaluated by surgical specimen | | | | | | |

### (2) Extraction of RNA

The above-mentioned frozen tissue samples were disrupted with Multi-beads Shocker^{®} (Yasui Kikai Corporation) while cooling the samples in liquid nitrogen to give powder. From the obtained powder, total RNA including miRNA was extracted with the miRNeasy Mini kit (QIAGEN).

### (3) Analysis by microarray technique

RNA (100 ng) extracted from each frozen tissue sample was processed with the Agilent miRNA Labeling Kit (Agilent Technologies) in order to add a fluorescence-labelled cytosine residue at the 3' end of miRNA in the RNA. The labelled RNA was added to the Human miRNA microarray Rel.12.0 (Agilent Technologies) and hybridization was carried out according to the protocol attached to the array. The fluorescence signal intensities were measured on the Microarray Scanner System G20505C (Agilent Technologies).

### (4) Analysis by quantitative RT-PCR

RNA (10 ng) extracted from each frozen tissue sample as described above was processed with the TaqMan^{®} MicroRNA Reverse Transcription Kit (Applied Biosystems) and a corresponding TaqMan^{®} MicroRNA Assay (Applied Biosystems) for one of 6 miRNAs in order to synthesize cDNA from the miRNA in the RNA and carry out qRT-PCR. qRT-PCR was carried out on the 7300 Real Time PCR System (Applied Biosystems). The kits mentioned above contain, for the respective 6 miRNAs including miRNA-100 and miRNA-125b, specific primers and TaqMan^{®} probes required for reverse transcription and real-time PCR. qRT-PCR was specifically carried out according to the protocol attached to the kits. The expression levels of the miRNAs were normalized with the expression level of a transcript of a small RNA, RNU6B (Applied Biosystems).

### (5) Analysis results

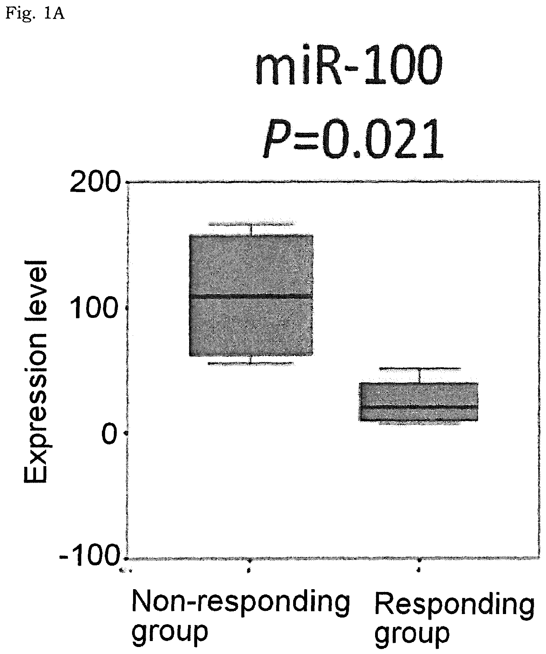

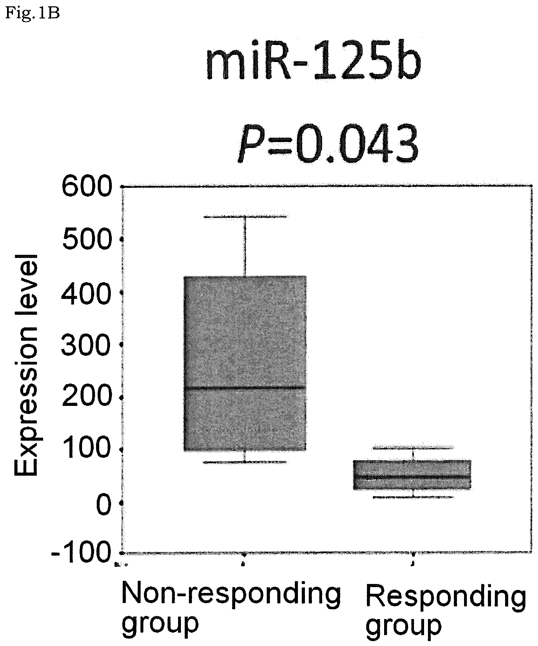

The microarray analysis identified 6 miRNAs including miRNA-100 and miRNA-125b of which expression levels were significantly different between the group of patients who responded to the preoperative chemotherapy and the group of patients who did not respond to the preoperative chemotherapy. The qRT-PCR analysis indicated that among 6 identified miRNAs, miRNA-100 and miRNA-125b had the expression levels which were significantly higher in the group of patients who did not respond to the preoperative chemotherapy than in the group of patients who responded to the preoperative chemotherapy (see Figs. 1A and 1B). These qRT-PCR analysis results are in conformity with the microarray analysis results.

### Example 2: Validation of expression of novel biomarkers

### (1) Preparation of biological samples

Twenty osteosarcoma patients different from those in Example 1 after receiving preoperative chemotherapy were divided into, according to the Huvos Grading System, a group of patients who responded to the preoperative chemotherapy (11 patients with Case Nos. 1 to 11) and a group of patients who did not respond to the preoperative chemotherapy (9 patients with Case Nos. 12 to 20) (see Table 2). Anticancer agents (neo-adjuvants) used in the preoperative chemotherapy were methotrexate, doxorubicin and cisplatin. In Example 2, the biological samples used were FFPE samples previously obtained from the 20 osteosarcoma patients by biopsy before the start of the preoperative chemotherapy. The group of patients who responded to the preoperative chemotherapy corresponded to the Huvos grade 1 or 2 and had the viability of osteosarcoma cells after the preoperative chemotherapy of less than 10% as measured by pathological examination of specimens obtained during operations. The group of patients who did not respond to the preoperative chemotherapy corresponded to, except for a fraction of patients, the Huvos grade 3 or 4 and had the viability of osteosarcoma cells after the preoperative chemotherapy of at or higher than 10% as measured by pathological examination of specimens obtained during operations. Viability of osteosarcoma cells for a fraction of patients in the group of patients who did not respond to the preoperative chemotherapy was determined by image analysis using CT and MRI.

**Table 2**

| **Case No.** | **Gender** | **Age** | **Location** | **Histologic type** | **neo-adjuvant** | **Viable cells (%)^{A}** |
|---|---|---|---|---|---|---|
| No. 1 | male | 19 | tibia | osteoblastic | MTX + DOX + CDDP | 0-10% |
| No. 2 | female | 9 | femur | osteoblastic | MTX + DOX + CDDP | 0% |
| No. 3 | female | 9 | tibia | chondroblastic | MTX + DOX + CDDP | 0-10% |
| No. 4 | female | 14 | femur | osteoblastic | MTX + DOX + CDDP | 0 |
| No. 5 | female | 15 | femur | osteoblastic | MTX + DOX + CDDP | 0-10% |
| No. 6 | female | 9 | tibia | osteoblastic | MTX + DOX + CDDP | 0% |
| No. 7 | male | 17 | femur | osteoblastic | MTX + DOX + CDDP | 0-10% |
| No. 8 | female | 17 | tibia | osteoblastic | MTX + DOX + CDDP | 1% |
| No. 9 | female | 11 | femur | chondroblastic | MTX + DOX + CDDP | 7% |
| No. 10 | male | 13 | femur | osteoblastic | MTX + D OX + CDDP | 0-10% |
| No. 11 | male | 16 | femur | chondroblastic | MTX + DOX + CDDP | 0-10% |
| No. 12 | female | 13 | tibia | osteoblastic | MTX + DOX + CDDP | 60-70% |
| No. 13 | male | 22 | femur | osteoblastic | MTX + DOX + CDDP | 30% |
| No. 14 | male | 18 | femur | osteoblastic | MTX + DOX + CDDP | 60-70% |
| No. 15 | male | 14 | tibia | osteoblastic | MTX + DOX + CDDP | 50-60% |
| No. 16 | male | 13 | femur | osteoblastic | MTX + DOX + CDDP | 30-40% |
| No. 17 | male | 9 | femur | chondroblastic | MTX + DOX + CDDP | 30-40% |
| No. 18 | female | 10 | femur | chondroblastic | MTX + DOX + CDDP | 70% |
| No. 19 | male | 17 | ilium | fibroblastic | MTX + DOX + CDDP | Clinically poor^{B} |
| No. 20 | male | 15 | tibia | osteblastic | MTX + DOX + CDDP | 50-60% |

| | | | | | | |
|---|---|---|---|---|---|---|
| A: Probability of viable cells were pathologically evaluated by surgical specimen B: Patients with disease progression in diagnostic imaging by computed tomography and magnetic resorce imaging | | | | | | |

### (2) Extraction of RNA

The above-mentioned FFPE tissue samples were sliced to obtain sections with the thickness of 10 µm. More than one section was placed in a 1.5-mL tube and total RNA including miRNA was extracted with the miRNeasy FFPE kit (QIAGEN).

### (3) Quantitative RT-PCR

RNA (10 ng) extracted from each FFPE tissue sample as described above was processed with the TaqMan^{®} MicroRNA Reverse Transcription Kit, the TaqMan^{®} MicroRNA Assay hsa-miR100 and the TaqMan^{®} MicroRNA Assay hsa-miR125b (all from Applied Biosystems) in order to synthesize cDNA from miRNA in the RNA and carry out qRT-PCR. qRT-PCR was carried out on the 7300 Real Time PCR System (Applied Biosystems). The above kits contain, for miRNA-100 and miRNA-125b, specific primers and TaqMan^{®} probes required for reverse transcription and real-time PCR. qRT-PCR was specifically carried out according to the protocol attached to the kits. The expression levels of miRNA-100 and miRNA-125b were normalized with the expression level of a transcript of a small RNA, RNU6B (Applied Biosystems).

### (4) Analysis results

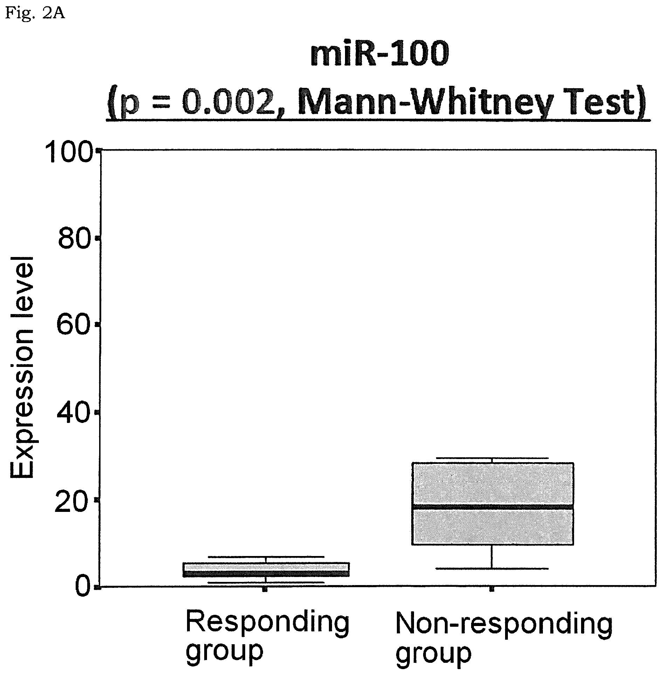

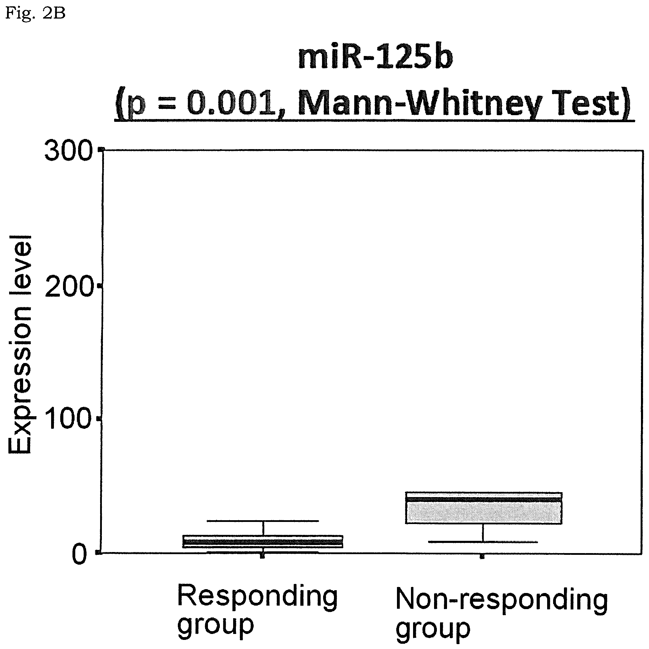

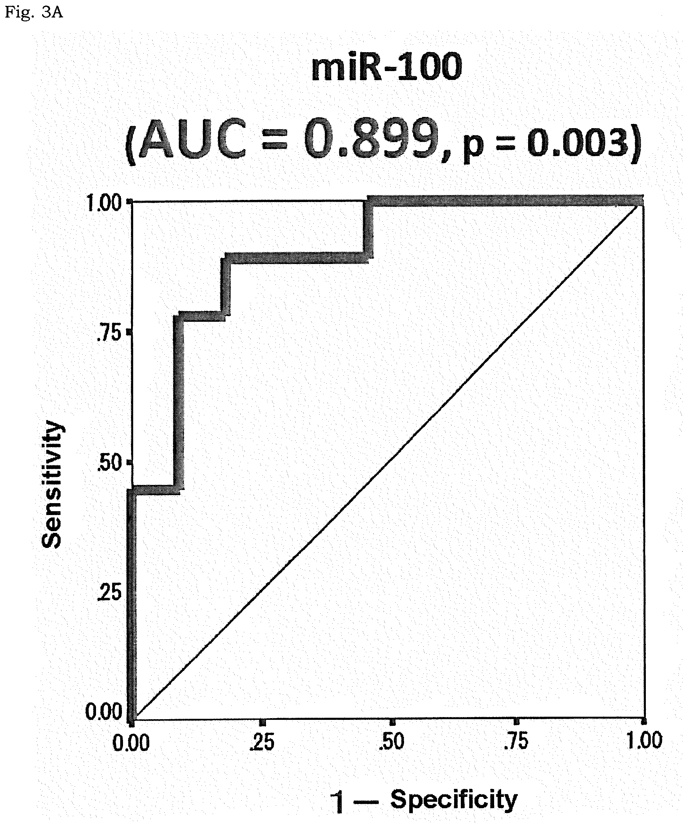

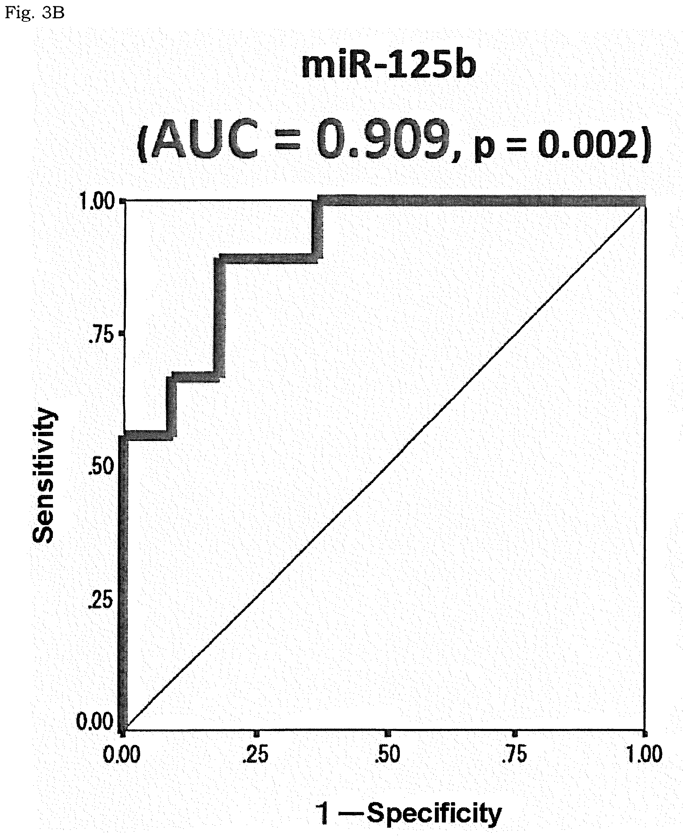

It was confirmed that even in patients different from those in Example 1, the expression levels of miRNA-100 and miRNA-125b were significantly higher in the groups of patients who did not respond to the preoperative chemotherapy than in the group of patients responded to the preoperative chemotherapy (see Figs. 2A and 2B). Based on the results of qRT-PCR, ROC curves were generated when sensitivity of the osteosarcoma patients to the preoperative chemotherapy was predicted, which are shown in Figs. 3A and 3B. These results show that based on the expression levels of miRNA-100 and miRNA-125b, sensitivity of osteosarcoma patients to an anticancer agent can be predicted with high accuracy before the start of treatment with the anticancer agent.

### Reference Example 1: Functional analysis of miRNA-100 and miRNA-125b

It was studied whether or not introduction of each of miRNA-100 and miRNA-125b into various osteosarcoma cell lines changes the sensitivity of the cells to anticancer agents.

### (1) Preparation of samples

Human osteosarcoma cell lines used were MNNG/HOS, MG63 and 143B. The cells were seeded in a 96-well plate at 3 x 10³ cells/well. After 24 hours, the medium was replaced with fresh medium and miRNA-100 (SEQ ID NO: 1), miRNA-125b (SEQ ID NO: 2) or negative control (nonsense RNA) (microRNA control, Non-target RNA (S10CM0600; COSMO BIO Co., Ltd)) was introduced to the cells using DharmaFECT transfection reagents (Thermo Scientific). At 24 hours after RNA introduction, the medium was replaced with fresh medium. Specific procedures followed the protocol attached to the gene transfer reagents used.

### (2) Sensitivity test

Methotrexate (MTX), doxorubicin (DOX) and cisplatin (CDDP) were applied to the cells lines MNNG/HOS, MG63 or 143B to which various RNAs were introduced under following conditions followed by incubation over a certain period. The viability of the cells thereafter was measured with the Cell Counting Kit-8 (Dojindo Laboratories). Specific procedures followed the protocol attached to the kit used.

Methotrexate (MTX): applied at a final concentration of 0 to 32 µM and incubated for 72 hours.

Doxorubicin (DOX): applied at a concentration of 0 to 1.3 µM and incubated for 48 hours.

Cisplatin (CDDP): applied at a final concentration of 0 to 32 µM and incubated for 48 hours.

### (3) Results

From the results of measurement of the viability of the cells, the 50% inhibitory concentration of cell proliferation (IC₅₀) for each anticancer agent was determined. The results are shown in Table 3.

**Table 3**

| | IC₅₀ | control | miR-125b | miR-100 |
|---|---|---|---|---|
| MNNG/HOS | MTX (µM) | 1.05 | 4.03* | 2.52* |
| | DOX (µM) | 0.18 | 0.37* | 0.25^{NS} |
| | CDDP (µM) | 5.70 | 7.32* | 6.97* |
| | | | | |
| MG63 | MTX (µM) | 3.09 | 7.74* | 7.18* |
| | DOX (µM) | 0.065 | 0.074* | 0.073* |
| | CDDP (µM) | 2.91 | 1.63* | 2.57* |
| | | | | |
| 143B | MTX (µM) | 1.49 | 3.97* | 3.21* |
| | DOX (µM) | 0.064 | 0.072* | 0.077* |
| | CDDP (µM) | 12.31 | 14.07* | 14.52* |

| | | | | |
|---|---|---|---|---|
| * p <0.05 NS: No significant difference | | | | |

From Table 3, it was found that cells to which miRNA-100 or miRNA-125b was introduced had an increased IC₅₀ for all three anticancer agents compared to osteosarcoma cells to which the negative control was introduced. These results suggested that miRNA-100 and miRNA-125b may confer resistance to anticancer agents on osteosarcoma cells.

### Reference Example 2: Effect of anticancer agent on miRNA-125b expression analyzed based on the expression amount of proapoptotic factor

BAK1 is a proapoptotic factor and it is known that expression thereof is suppressed by an increase in expression level of miRNA-125b. In Reference Example 2, effect of an anticancer agent on expression of miRNA-125b was studied based on the expression level of BAK1 in osteosarcoma cells.

### (1) Preparation of samples

A human osteosarcoma cell line used was MNNG/HOS. The cells were seeded in a 96-well plate at 3 x 10³ cells/well. After 24 hours, the medium was replaced with fresh medium and miRNA-125b (SEQ ID NO: 2) or negative control (nonsense RNA) (microRNA control, Non-target RNA (S10CM0600; COSMO BIO Co., Ltd)) was introduced to the cells using DharmaFECT transfection reagents (Thermo Scientific). At 24 hours after RNA introduction, the medium was replaced with fresh medium. Specific procedures followed the protocol attached to the gene transfer reagents used.

### (2) Measurement of the expression level of BAK1 in MNNG/HOS cells

### (i) Extraction of protein from cells

Doxorubicin (DOX) at a final concentration of 0 µM or 64 µM was applied to MNNG/HOS cells to which various RNAs were introduced as above (1) and then the cells were incubated for 48 hours. To the collected cells was added a urea-containing lysis buffer and the mixture was incubated on ice for 30 minutes to lyse the cells. The lysed cells were centrifuged at 15,000 rpm for 30 minutes at 4°C and the supernatant was collected. The protein concentration in the supernatant was quantified by the Bradford assay (Bio-Rad).

### (ii) Detection of BAK1 protein by western blotting

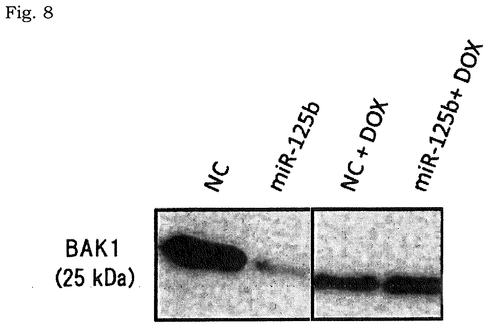

Proteins in the supernatant were separated by electrophoresis using an SDS/polyacrylamide gel and transferred onto a nitrocellulose membrane (Bio-Rad). Thereafter, the membrane was blocked for 1 hour in a solution (hereinafter also referred to as "blocking buffer") of 5% skimmed milk powder in Tris-buffered saline containing Tween 20 (TBS-T). After the blocking, the membrane was incubated overnight at 4°C in a solution of a primary antibody (Anti-BAK1 polyclonal antibody (MBL)) diluted to 1:250 in the blocking buffer. Thereafter the membrane was thoroughly washed with TBS-T and incubated in a solution of a horseradish peroxidase-labelled anti-rabbit secondary antibody (GE) diluted to 1:2000 in the blocking buffer. After the membrane was washed with TBS-T, the antigen-antibody complex was detected with the ECL-Prime kit (GE). The results are shown in Fig. 8.

As shown in Fig. 8, without application of DOX, the expression level of BAK1 in the negative control-introduced cell line (NC) was clearly different from that in the miRNA-125b-introduced cell line. On the other hand, with application of DOX, the expression level of BAK1 in NC was almost the same as that in the miRNA-125b-introduced cell line. Given the results of Reference Example 2 and the knowledge that expression of BAK1 is suppressed by an increase in the expression level of miRNA-125b, it is believed that application of DOX may affect expression level of miRNA-125b in osteosarcoma cells. Therefore it was suggested that in order to avoid any action of an anticancer agent such as DOX on expression level of miRNA-125b, the expression level of miRNA-125b is preferably measured before administration of the anticancer agent to a patient.

## Claims

1. A method for predicting sensitivity of an osteosarcoma patient to an anticancer agent, comprising the steps of:
extracting RNA from a biological sample containing osteosarcoma cells collected from the osteosarcoma patient to whom the anticancer agent is not administered;
obtaining a value indicating an expression level of at least one of miRNA-100 and miRNA-125b in the extracted RNA; and
based on the obtained value indicating the expression level, predicting sensitivity of the osteosarcoma patient to the anticancer agent;
wherein in the step of predicting, the value indicating the expression level is compared with a predetermined threshold and sensitivity of the osteosarcoma patient to the anticancer agent is predicted based on the comparison result;
wherein in the step of predicting, when the value indicating the expression level is at or higher than the predetermined threshold, it is predicted that sensitivity of the osteosarcoma patient to the anticancer agent is low and, when the value indicating the expression level is less than the predetermined threshold, it is predicted that sensitivity of the osteosarcoma patient to the anticancer agent is high.

2. The method according to claim 1, wherein the biological sample is a cell, tissue or blood collected from the osteosarcoma patient to whom the anticancer agent is not administered.

3. The method according to any one of claims 1 to 2,
wherein in the step of predicting, sensitivity to at least one anticancer agent selected from an anti-metabolite, an anticancer antibiotic, a platinum agent, an anticancer plant alkaloid and an alkylating agent is predicted.

4. The method according to claim 3, wherein the anti-metabolite is at least one selected from methotrexate, tegafur, carmofur, 5-fluorouracil, cytarabine, gemcitabine, capecitabine, fludarabine, pemetrexed, pentostatin and enocitabine.

5. The method according to claim 3 or 4, wherein the anticancer antibiotic is at least one selected from doxorubicin, daunorubicin, bleomycin, aclarubicin, idarubicin, epirubicin, pirarubicin, peplomycin, mitoxantrone, actinomycin D and mitomycin C.

6. The method according to any one of claims 3 to 5,
wherein the platinum agent is at least one selected from cisplatin, carboplatin, oxaliplatin and nedaplatin.

7. The method according to any one of claims 3 to 6,
wherein the anticancer plant alkaloid is at least one selected from docetaxel, paclitaxel, irinotecan, nogitecan, etoposide, sobuzoxane, vinorelbine, vindesine, vincristine and vinblastine.

8. The method according to any one of claims 3 to 7,
wherein the alkylating agent is at least one selected from ifosfamide, cyclophosphamide, melphalan, busulfan, nimustine, temozolomide and dacarbazine.

## Patentansprüche

1. Verfahren zum Vorhersagen der Sensibilität eines Osteosarkompatienten gegenüber einem Antikrebsmittel, wobei das Verfahren die Schritte umfasst:
Extrahieren von RNA aus einer biologischen Probe, die Osteosarkomzellen enthält, die von dem Osteosarkompatienten gewonnen wurden, dem das Antikrebsmittel nicht verabreicht wird;
Erhalten eines Wertes, der ein Expressionsniveau wenigstens einer von miRNA-100 und miRNA-125b in der extrahierten RNA anzeigt; und
auf Basis des erhaltenen Wertes, der das Expressionsniveau anzeigt, Vorhersagen der Sensibilität des Osteosarkompatienten gegenüber dem Antikrebsmittel;
wobei in dem Schritt des Vorhersagens der Wert, der das Expressionsniveau anzeigt, mit einem vorbestimmten Schwellwert verglichen wird und die Sensibilität des Osteosarkompatienten gegenüber dem Antikrebsmittel auf Basis des Vergleichsergebnisses vorhergesagt wird;
wobei in dem Schritt des Vorhersagens, wenn der Wert, der das Expressionsniveau anzeigt, auf dem vorbestimmten Schwellwert liegt oder höher ist, vorhergesagt wird, dass die Sensibilität des Osteosarkompatienten gegenüber dem Antikrebsmittel gering ist, und, wenn der Wert, der das Expressionsniveau anzeigt, kleiner als der vorbestimmte Schwellwert ist, vorhergesagt wird, dass die Sensibilität des Osteosarkompatienten gegenüber dem Antikrebsmittel hoch ist.

2. Verfahren gemäß Anspruch 1, wobei die biologische Probe eine Zelle, Gewebe oder Blut ist, gewonnen von dem Osteosarkompatienten, dem das Antikrebsmittel nicht verabreicht wird.

3. Verfahren gemäß irgendeinem der Ansprüche 1 bis 2, wobei in dem Schritt des Vorhersagens die Sensibilität gegenüber wenigstens einem Antikrebsmittel, ausgewählt unter einem Antimetaboliten, einem Antikrebs-Antibiotikum, einem platinhaltigen Mittel, einem Antikrebs-Pflanzenalkaloid und einem Alkylierungsmittel, vorhergesagt wird.

4. Verfahren gemäß Anspruch 3, wobei der Antimetabolit wenigstens einer, ausgewählt unter Methotrexat, Tegafur, Carmofur, 5-Fluoruracil, Cytarabin, Gemcitabin, Capecitabin, Fludarabin, Pemetrexed, Pentostatin und Enocitabin, ist.

5. Verfahren gemäß Anspruch 3 oder 4, wobei das Antikrebs-Antibiotikum wenigstens eines, ausgewählt unter Doxorubicin, Daunorubicin, Bleomycin, Aclarubicin, Idarubicin, Epirubicin, Pirarubicin, Peplomycin, Mitoxantron, Actinomycin D und Mitomycin C, ist.

6. Verfahren gemäß irgendeinem der Ansprüche 3 bis 5, wobei das platinhaltige Mittel wenigstens eines, ausgewählt unter Cisplatin, Carboplatin, Oxaliplatin und Nedaplatin, ist.

7. Verfahren gemäß irgendeinem der Ansprüche 3 bis 6, wobei das Antikrebs-Pflanzenalkaloid wenigstens eines, ausgewählt unter Docetaxel, Paclitaxel, Irinotecan, Nogitecan, Etoposid, Sobuzoxan, Vinorelbin, Vindesin, Vincristin und Vinblastin, ist.

8. Verfahren gemäß irgendeinem der Ansprüche 3 bis 7, wobei das Alkylierungsmittel wenigstens eines, ausgewählt unter Ifosfamid, Cyclophosphamid, Melphalan, Busulfan, Nimustin, Temozolomid und Dacarbazin, ist.

## Revendications

1. Procédé pour prévoir la sensibilité d'un patient atteint d'ostéosarcome à un agent anticancéreux, comprenant les étapes de :
extraction d'ARN provenant d'un échantillon biologique contenant des cellules d'ostéosarcome recueillies auprès du patient atteint d'ostéosarcome à qui l'agent anticancéreux n'est pas administré ;
obtention d'une valeur indiquant un niveau d'expression d'au moins un de miARN-100 et miARN-125b dans l'ARN extrait; et
sur la base de la valeur obtenue indiquant le niveau d'expression, prédiction de la sensibilité du patient atteint d'ostéosarcome à l'agent anticancéreux ;
dans lequel dans l'étape de prédiction, la valeur indiquant le niveau d'expression est comparée à un seuil prédéterminé et la sensibilité du patient atteint d'ostéosarcome à l'agent anticancéreux est prédite sur la base du résultat de comparaison ;
dans lequel dans l'étape de prédiction, quand la valeur indiquant le niveau d'expression est au niveau du seuil prédéterminé ou plus élevée, il est prédit que la sensibilité du patient atteint d'ostéosarcome à l'agent anticancéreux soit basse et, lorsque la valeur indiquant le niveau d'expression est inférieure au seuil prédéterminé, il est prévu que la sensibilité du patient atteint d'ostéosarcome à l'agent anticancéreux soit élevée.

2. Procédé selon la revendication 1, dans lequel l'échantillon biologique est une cellule, du tissu ou du sang recueilli auprès du patient atteint d'ostéosarcome à qui l'agent anticancéreux n'est pas administré.

3. Procédé selon l'une quelconque des revendications 1 à 2, dans lequel dans l'étape de prédiction, la sensibilité à au moins un agent anticancéreux sélectionné parmi un antimétabolite, un antibiotique anticancéreux, un agent au platine, un alcaloïde végétal anticancéreux et un agent alkylant est prédite.

4. Procédé selon la revendication 3, dans lequel l'antimétabolite est au moins un sélectionné parmi méthotrexate, tégafur, carmofur, 5-fluoro-uracile, cytarabine, gemcitabine, capécitabine, fludarabine, pémétrexed, pentostatine et énocitabine.

5. Procédé selon la revendication 3 ou 4, dans lequel l'antibiotique anticancéreux est au moins un sélectionné parmi doxorubicine, daunorubicine, bléomycine, aclarubicine, idarubicine, épirubicine, pirarubicine, péplomycine, mitoxantrone, actinomycine D et mitomycine C.

6. Procédé selon l'une quelconque des revendications 3 à 5, dans lequel l'agent au platine est au moins un sélectionné parmi cisplatine, carboplatine, oxaliplatine et nédaplatine.

7. Procédé selon l'une quelconque des revendications 3 à 6, dans lequel l'alcaloïde végétal anticancéreux est au moins un sélectionné parmi docétaxel, paclitaxel, irinotécan, nogitécan, étoposide, sobuzoxane, vinorelbine, vindésine, vincristine et vinblastine.

8. Procédé selon l'une quelconque des revendications 3 à 7, dans lequel l'agent alkylant est au moins un sélectionné parmi ifosfamide, cyclophosphamide, melphalan, busulfan, nimustine, témozolomide et dacarbazine.


### SEQUENCE LISTING

<110> National Cancer Center SYSMEX CORPORATION
<120> METHOD FOR PREDICTING SENSITIVITY OF OSTEOSARCOMA PATIENT TO ANTICANCER AGENT
<130> TM5866EP
<150> JP2014-074142
   <151> 2014-03-31
<150> JP2015-007991
   <151> 2015-01-19
<160> 2
<170> PatentIn version 3.5
<210> 1
   <211> 22
   <212> RNA
   <213> Homo sapiens
<400> 1
   aacccguaga uccgaacuug ug 22
<210> 2
   <211> 22
   <212> RNA
   <213> Homo sapiens
<400> 2
   ucccugagac ccuaacuugu ca 22